# EUROPEAN PATENT SPECIFICATION

(11) **EP 3 641 796 B1**
(45) Date of publication and mention of the grant of the patent: **14.10.2026**
(21) Application number: 18737809.6
(22) Date of filing: 18.06.2018
(51) Int. Cl.: A61K 35/747, A61P 1/00, A61P 3/00, A61P 17/00, A61P 5/00

(54) **THERAPEUTIC USES OF LACTOBACILLUS PLANTARUM**
THERAPEUTISCHE VERWENDUNG VON LACTOBACILLUS PLANTARUM
UTILISATIONS THÉRAPEUTIQUES DE LACTOBACILLUS PLANTARUM

(30) Priority: 19.06.2017 GB 201709763
(43) Date of publication of application: 29.04.2020
(73) Proprietor: Probi AB, 223 70 Lund (SE)
(72) Inventor: ROBERTSON, Anna-Karin, 223 70 Lund (SE)
(74) Representative: Potter Clarkson
(86) International application number: PCT/EP2018/066152
(87) International publication number: WO 2018/234255

(56) References cited:
- WO-A1-2007/036230
- WO-A1-2007/040444
- WO-A1-2016/149687
- WO-A1-99/07827
- WO-A2-2008/105715
- NIEDZIELIN K ET AL: "A CONTROLLED, DOUBLE-BLIND, RANDOMIZED STUDY ON THE EFFICACY OF LACTOBACILLUS PLANTARUM 299V IN PATIENTS WITH IRRITABLE BOWEL SYNDROME", EUROPEAN JOURNAL OF GASTROENTEROLOGY HEPATOLOGY, XX, XX, vol. 13, no. 10, 1 October 2001 (2001-10-01), pages 1143 - 1147, XP001119326, DOI: 10.1097/00042737-200110000-00004
- DUCROTTÉ P ET AL: "Clinical trial: Lactobacillus plantarum 299v (DSM 9843) improves symptoms of irritable bowel syndrome", WORLD JOURNAL OF GASTROENTEROLOGY, WJG PRESS, CN, vol. 18, no. 30, 14 August 2012 (2012-08-14), pages 4012 - 4018, XP002697271, ISSN: 1007-9327, [retrieved on 20120814], DOI: 10.3748/WJG.V18.I30.4012

## Description

The present invention relates to at least one strain of *Lactobacillus plantarum* DSM 9843 for increasing the numbers of Oscillospira spp. in a subject, for use in the treatment of abdominal pain in a subject suffering from irritable bowel syndrome (IBS), wherein the subject has a decrease level of Oscillospira spp.

### Background

"Dysbiosis" is a term for a deleterious imbalance or disturbance in the normally diverse microbiota (microbial population) of the gastrointestinal tract (GI). It is known that dysbiosis of the microbiota can lead to a wide variety of deleterious conditions and/or diseases.

Dysbiosis of Oscillospira spp., involves decreased levels of Oscillospira spp., and is associated with several undesirable conditions, disorders, syndromes and/or diseases, including gastrointestinal disorders, metabolic disorders and/or inflammatory disorders or diseases, such as obesity, high BMI (Tims et al., ISME J. (April 2013); 7(4): 707-717. doi:10.1038/ismej.2012.146.Epub 2012 Nov 29.) and type 2 diabetes; autism and pervasive development disorder (De Angelis et al., (2013) PLOS ONE 8(10):e76993); flatulence (Manichanh et al., Gut (2014); 63:401-408); intestinal permeability (Hamilton et al., AM J. Physiology (2015); 308 (10): G840-G851; and Lam et al., (2012) PLOS ONE 7(3):e34233); Irritable Bowel Syndrome (IBS); and other inflammatory-related disorders, such as Inflammatory Bowel Disease (IBD) including Crohn's disease (Kaakoush et al., J. Clinical Microbiology (2012); 50(10):3258-3266), and/or ulcerative colitis; childhood metabolic disease and/or atopic disease; atherosclerosis; non-alcoholic fatty liver disease; dyslipidemia; and travellers' diarrhoea.

WO2016/149687 discloses that administration of a daily dose of 10 billion CFU (10¹⁰ CFU) Lactobacillus plantarum 299v (DSM 9843) improves abdominal pain in patient suffering from irritable bowel syndrome (IBS).

Tun et al., Microbiome (2017) 5:40 investigated measures for reducing the risk to health of overweight (obesity) and allergic disease. They showed that increased abundance of Oscillospira is caused by pre-and/or post-natal exposure to furry pets. They also noted that increased abundance of Oscillospira is associated with leanness or lower body mass index (BMI) in both infants and adults. Thus, increased Oscillospira could be the mechanism by which exposure to furry pets reduced the risk for childhood metabolic and/or atopic disease. Conversely, they noted that decreasing abundance of Oscillospira with obesity has been reported, in three different geographical regions, despite substantial differences in gut microbial composition.

Atherosclerosis is the major cause of myocardial infarction and stroke, which is the leading cause of death worldwide. Atherosclerosis is a chronic inflammatory disease of the arteries.

Chan et al., BMC Microbiology (2016) 16:264 studied high fat diet-induced atherosclerosis in mice and found that reduced Oscillospira abundance was associated with a significant adverse atherogenic profile, especially atherosclerotic plaque size and increased plasma levels of adipocyte-fatty acid binding protein and cholesterol. They also concluded that Oscillospira are most likely protective against atherosclerosis.

According to Tun et al., Microbiome (2017)5:40, the health-promoting effects of Oscillospira are not fully understood, but they are thought to produce butyrate by relying on fermentation products secreted by other bacterial species or on sugars liberated from host mucins.

Butyrate appears to be an important signalling molecule with other potential health benefits, including metabolic health. Acetate, propionate and butyrate are the three most abundant short chain fatty acids (SCFA) detected in the colon.

Butyrate is produced in the human intestine by certain commensal bacteria. At the intestinal level, butyrate plays a regulatory role on the transepithelial fluid transport, ameliorates mucosal inflammation and oxidative status, reinforces the epithelial defense barrier, and modulates visceral sensitivity and intestinal motility (Canani et al., World J. Gastroenterol (2011, March 28); 17(12):1519-1528). In addition, a growing number of studies have stressed the role of butyrate in the prevention and inhibition of colorectal cancer. The anti-inflammatory effect of butyrate may depend on the inhibition of proinflammatory macrophages and dendritic cells and the promotion of regulatory T cell generation (Arpaia et al., Nature (19/26 Dec 2013) vol 504:451-455; Chang et al., P.N.A.S (Feb 11, 2014) vol 111(6):2247-2252; and Singh et al., Immunity (Jan 16, 2014); 40(1):128-139).

At the systemic level, butyrate exerts potentially useful effects on many conditions, including hemoglobinopathies, genetic metabolic diseases, hypercholesterolemia, insulin resistance, and ischemic stroke (Canani et al., World J. Gastroenterol (2011, March 28); 17(12):1519-1528).

Butyrate-producers are in lower abundance in people with metabolic disease, such as overweight, metabolic syndrome, insulin resistance, type II diabetes and dyslipidemia. Butyrate increases GLP-1 in L-cells, and may this play an active role in the control of blood glucose and help prediabetic subjects maintain normal blood glucose concentration/reduce post-prandial glycemic responses. (Yadav et al., J. Biol. Chem (Aug 30, 2013); vol 288 (35):25088-25097; Qin et al., Nature (Oct 4, 2012); vol 490:55-60; Karlsson et al., Nature (June 6, 2013); vol 498:99-103; and Vrieze et al., Gastroenterology (2012); 143:913-916).

Butyrate-producing probiotic bacteria (Clostridium butyricum Miyairi 588) have been shown to reduce choline-deficient/L-amino acid-defined -diet-induced hepatic lipid deposition (non-alcoholic fatty liver disease) in rodents and significantly improved the triglyceride content, insulin resistance, serum endotoxin levels, and hepatic inflammatory indexes (Endo et al., PLOS ONE (May 2013); vol 8(5):e63388. doi:10.1371/journal.pone.0063388).

Krokowicz et al., Travel Med. Infect. Dis (Mar-Apr, 2014); 12(2):183-8 showed that administration of sodium butyrate and short-chain fatty acids decreased the occurrence of travellers' diarrhoea and may constitute a new method of travellers' diarrhoea prevention. Since increasing Oscillospira spp. numbers in accordance with the present invention can be expected to increase butyrate production, the methods uses and kits of the present invention may also find utility in the prevention and/or treatment of travellers' diarrhoea.

Hence, butyrate-producing bacteria have the potential to treat or prevent metabolic disease and/or inflammatory conditions through several different mechanisms of action (Brahe et al., Int. Ass. For the Study of Obesity (2013):1-9. doi:10.1111/obr.12068).

Hence, there is a need to increase levels of Oscillospira spp. in a subject to obtain and preferably maintain the beneficial health benefits associated therewith. In particular, there is a need to provide materials and methods for use in the treatment and/or prophylaxis of a dysbiosis of Oscillospira spp. However, a particular problem in developing effective treatments is that Oscillospira is an anaerobic bacterial genus from Clostridial cluster IV that has resisted cultivation for over a century since the first time it was observed (see Gophna et al., Environmental Microbiology (2017) 19(3), 835-841). Accordingly, it is not possible to culture, prepare and administer Oscillospira spp. to a subject in order to increase their abundance.

### Summary of the Invention

The invention relates to at least one strain of Lactobacillus plantarum DSM 9843 for use in the treatment of abdominal pain in a subject suffering from irritable bowel syndrome (IBS), wherein the subject is characterised by having a decreased number of Oscillospira spp. in a test sample derived from said subject relative to a control sample derived from a healthy subject or from a pool of samples from healthy subjects, wherein the Lactobacillus plantarum DSM 9843 is administered in an amount of from 10⁸ - 10¹¹ CFU per daily dose, preferably 10¹⁰ CFU per daily dose to attain increased numbers of Oscillospira spp. in the subject.

The treatment and/or prophylaxis with at least one strain of *Lactobacillus plantarum* DSM 9843 is effective to increase and preferably maintain increased numbers of Oscillospira spp. in a subject compared to Oscillospira spp. numbers without treatment.

By "increased numbers of Oscillospira spp." we include the meaning that the total number of Oscillospira bacteria of any and all Oscillospira species is increased in the subject, or a relevant sample therefrom.

### Detailed Description of the Invention

In the description of the invention various embodiments and/or individual components are disclosed. As will be apparent to the ordinarily skilled practitioner, all combinations of such embodiments and components taught in the disclosure are possible and can result in preferred embodiments of the present invention.

Any percentages and ratios are calculated by weight unless otherwise indicated. All percentages, parts and ratios are calculated based on the total composition unless otherwise indicated.

Referenced herein may be trade names for components including various ingredients utilized in the present invention. However, the inventors do not intend to be limited by materials under a certain trade name. Equivalent materials (eg those obtained from a different source under a different name or reference number) to those referenced by trade name may be substituted and used in the embodiments of the invention described herein.

### Lactobacillus plantarum

The at least one strain *Lactobacillus plantarum* DSM 9834 for use according to the invention may be and/or dead. Preferably, the strain is a probiotic, that is, a viable strain which, upon administration, confers a health benefit to a recipient mammal, preferably a human. The composition may comprise a single species or strain of said probiotic bacterium, or it may comprise a combination of one or more species or strains.

Probiotic *Lactobacillus plantarum* strains are selected from one or more of *Lactobacillus plantarum* 299v (DSM 9843, Deposited on 16/03/1995); *Lactobacillus plantarum* 299 (DSM 6595, Deposited on 02/07/1991); *Lactobacillus plantarum HEAL* 9 (DSM 15312, Deposited on 27/11/2002); *Lactobacillus plantarum* HEAL 19 (DSM 15313, Deposited on 27/11/2002); *Lactobacillus plantarum* HEAL 99 (DSM 15316, Deposited on 27/11/2002); and *Lactobacillus plantarum* Gos 42 (DSM 32131, Deposited on 02/09/2015) which have been deposited by Probi AB of Sölvegatan 41 A, SE-223 701 Lund, Sweden at the depository DSMZ of Inhoffenstrasse 7B, 38124 Braunschweig, Germany in accordance with the provisions of the Budapest Treaty.

Other described probiotic *Lactobacillus plantarum* strains are *Lactobacillus plantarum* LB3e (DSM 17852 and *Lactobacillus plantarum* LB7c (DSM 17853, both deposited on 06/01/2006 by ProBac AB of Box 3049, Se-903 02 Umeå, Sweden); and *Lactobacillus plantarum LB931* (DSM 11918, deposited on 09/01/1998 by Essum AB (www.essum.se) of Nygatan 74, SE-903 02 Umeå, Sweden). The three strains were all deposited at DSMZ of Inhoffenstrasse 7B, 38124 Braunschweig, Germany.

The strain for use according to the invention is *Lactobacillus plantarum* 299v (DSM 9843).

*Lactobacillus plantarum* 299 (DSM 6595) was deposited on 2 July 1991 at DSM-DEUTSCHE SAMMLUNG VON MIKROORGANISMEN UND ZELLKULTUREN GmbH in the name of Probi.

*Lactobacillus plantarum* 299v (DSM 9843) was deposited on 16 March 1995 at DSM-DEUTSCHE SAMMLUNG VON MIKROORGANISMEN UND ZELLKULTUREN GmbH, Mascheroder Weg 1b, D-38124 Braunschweig, Germany, by Probi AB.

*Lactobacillus plantarum* HEAL 9 (DSM 15312), *Lactobacillus plantarum* HEAL 19 (DSM 15313), and *Lactobacillus plantarum* HEAL 99 (DSM 15316) were deposited on 27 November 2002 at DSMZ-DEUTSCHE SAMMLUNG VON MIKROORGANISMEN UND ZELLKULTUREN GmbH, Mascheroder Weg 1b, D-38124 Braunschweig, Germany, by Probi AB.

*Lactobacillus plantarum* GOS42 (DSM 32131) was deposited on 2 September 2015 at Leibniz Institute DSMZ-German Collection of Microorganisms and Cell Cultures, Inhoffenstr. 7 B, D-38124 Braunschweig, Germany by Probi AB.

*Lactobacillus plantarum* LB3e (DSM 17852) and *Lactobacillus plantarum* LB7c (DSM 17853) were deposited on 6 January 2006 at DSMZ-DEUTSCHE SAMMLUNG VON MIKROORGANISMEN UND ZELLKULTUREN GmbH, Mascheroder Weg 1b, D-38124 . Braunschweig, Germany, by Probac AB. All rights and duties in connection with deposits DSM 17852 and DSM 17853 were given to and accepted by Probi AB, which is now the depositor of said strains.

*Lactobacillus plantarum* LB931 (DSM 11918) was deposited on 9 January 1998 at DSMZ-DEUTSCHE SAMMLUNG VON MIKROORGANISMEN UND ZELLKULTUREN GmbH, Mascheroder Weg 1b, D-38124 Braunschweig, Germany, by Essum AB, and which was made available to the public by disclosure in granted patent EP1060240.

The viability of the probiotic bacteria may be confirmed by plating the bacteria on a suitable medium (e.g. solidified agar in a standard sized Petri dish) and counting the number of colonies formed. The measure, colony forming unit (or CFU), is used to quantify the amount of viable (live) bacteria in the composition.

Thus, the initial colony forming units (CFU) and the continued stability and viability of the composition partly depend on the amount of moisture in the composition. As described herein, the composition is packaged and stored in containers (preferably sealed container)

in order to provide oxygen and moisture barrier in order to protect the integrity of the probiotic bacteria in the composition.

The colony forming units (CFU) referred to in the context of the composition of the present invention are CFU in a single dose after the preparation of the composition.

### Effective amounts of Lactobacillus plantarum

The at least one strain of *Lactobacillus plantarum* DSM 9843 present in the compositions for use according to the invention at a CFU per dose of 10⁸ - 10¹¹ CFU/dose, most preferably 10¹⁰ CFU/dose.

### Lactobacillus-containing compositions

The Lactobacillus-containing compositions for use according to the invention may be provided in a variety of single and/or multi-dose formulations, including capsules, tablets and powders. Preferably, the compositions for use according to the invention are adapted for oral administration to a subject.

### Compositions and formulations

The probiotic strains for use according to the invention are preferably freeze-dried.

The probiotic strains for use according to the invention may be provided together with a suitable carrier, diluent or excipient as a solid or liquid formulation, which may be a pharmaceutical formulation in on embodiment.

Examples of a suitable liquid carrier include water and other aqueous solvents.

Examples of a suitable solid carrier include maltodextrin, inulin, potato starch, corn starch or other vegetable starch, microcrystalline cellulose (MCC), and sugar alcohols.

The composition may be a dry fermented or non-fermented composition. In the case of a dry non-fermented composition, fermentation takes place in the gastrointestinal tract after ingestion of the composition by a subject.

In use, the probiotic strain(s) for use according to the invention may be mixed with a liquid or solid carrier before administration to a subject. For example, the subject may mix the strain(s) with a carrier consisting of water or some other aqueous solvent, or a drink prior to intake. Similarly, the probiotic strains may be mixed with a carrier consisting of one or more foods. Preferred foods are gluten free products such as fermented or non-fermented dairy products such as yoghurts, fruit juices; beverages, soups, plant based foods such as soy products, dry food bars, baby food, infant nutrition, infant formula, breast milk replacements from birth.

Infant or baby formula milk is a particularly preferred carrier for the probiotic of *Lactobacilli* strain(s) of the invention. It may be in a dry powder form for mixing with water before feeding it to babies as a ready-to-feed liquid form. It is normally made from cows' milk and contains whey and casein protein.

The probiotic strain(s) for use according to the invention may also be provided in a composition together with one or more ingredients of known dietary supplements, for example, micronutrients such as vitamins and minerals.

### Packaging

The compositions for use according to the present invention are typically filled in a sealed container, which provides an oxygen and moisture barrier in order to protect and maintain the viability of any live bacteria in the composition.

Preferably the composition is packaged in sealed aluminium foil sticks, where each stick comprises one dose of the composition, i.e. one dose of the probiotic bacteria.

### Methods of administration and use

The *Lactobacillus plantarum-containing* compositions for use according to the invention described herein are preferably orally administered, although other routes including rectal administration may be contemplated.

As used herein, the term "orally administering" with respect to the subject means that the mammal ingests or a human is directed to administer, or does administer, to oneself (or another human or other animal) one or more of the compositions herein. Where the human is directed to administer the composition, such direction can be that which instructs and/or informs the human that use of the composition may and/or will provide the referenced benefit, for example, alleviation of one or more symptoms associated with a disease or condition. For example, such direction may be oral direction (eg through oral instruction from, for example, a physician, pharmacist, nurse, or other health professional), radio or television media (ie advertisement), or written direction (eg through written direction from, for example, a health professional *(eg* scripts), sales professional or organization *(eg* through, for example, marketing brochures, pamphlets, written media (eg internet, electronic mail, or other computer-related media), and/or packaging associated with the composition (eg a label present on a container holding the composition). As used herein, "written" means through words, pictures, symbols, and/or other visible descriptors. Such information need not utilize the actual words used herein, for example, "human", or "treatment", but rather use of words, pictures, symbols, and the like conveying the same or similar meaning are contemplated within the scope of this invention.

Administration may be on an as-needed or as-desired basis in order to increase and preferably maintain increased numbers of Oscillospira spp., for example, once-monthly, once-weekly, or daily, including multiple times daily, to arrive at a total daily dose or amount of probiotic bacteria, whether administered every day, one day per week, one day per month, or on a given day as needed. The amount of composition utilized may be dependent on a variety of factors, including the health status of the subject mammal, age, gender, or other like factors of ordinary consideration.

Preferably, the subjected treated in accordance with the invention is mammalian, most preferably human, bovine, canine or feline; or avian, most preferably one or more species of poultry such as chickens or turkeys.

In a preferred embodiment the subject is a human selected from: a male or female infant between 0 - 6 months old; a male or female child from 6 months to 16 years old, especially 6 months - 6 years old; and a male or female adult.

The male or female adult may be from 16 - 65, or more than 65 years old. The human female adult may be of child-bearing age or be post-menopausal.

### Detection and enumeration of Oscillospira spp.

Preferably, numbers of Oscillospira spp. are detected in relevant biological samples. For example, samples can be taken from the rumen (fluid or whole rumen content samples) of herbivore mammals such as domestic cattle or sheep; faecal samples and/or samples can also be taken from appropriate regions of the GI tract of humans.

Suitable molecular techniques for detecting and enumerating Oscillospira spp. are described in Mackie et al, Applied and Environmental Microbiology, Nov 2003, p 6808-6815 and involve the use of PCR primers based on Oscillospira-specific 16S ribosomal RNA gene sequences.

In particular, Mackie *et al,* (2003) describe PCR and PCR-denaturing gradient gel electrophoresis (DGGE) procedures for the detection of Oscillospira spp. They used the techniques to determine the occurrence of this bacterium in different ruminants including cattle, sheep and reindeer.

Klindworth et al., Nucleic Acids Research vol. 41, No. 1 (published online 28 August 2012) evaluated general 16S ribosomal RNA gene PCR primers and provide guidelines for selecting suitable primer pairs for studying microbial levels and diversity for classical and next-generation sequencing-based studies.

The level of Oscillospira spp. in said test sample can be compared to a level of said Oscillospira spp. in a control sample. The control sample may advantageously be derived from a healthy subject or from a pool of samples from healthy subjects, and is preferably treated in the same way as is the test sample. Thus, preferably the control sample is sampled in the same way as is the test sample, if applicable, nucleic acid is isolated in the same way as is the test sample, and, if applicable, hybridization or quantitative amplification is performed under the same conditions to allow a fair comparison of the test sample and control sample. It is not necessary to determine the level of Oscillospira spp. in a control sample each time a test sample is measured; once the level is reliably determined in a control sample, the level values may be stored, e.g., in a computer, and used for the comparative purposes.

Other suitable methods of enumerating Oscillospira spp. in a subject are disclosed in US 2012/0238468, concerning determining and comparing the levels of Oscillospira spp. in samples from healthy and diseased subjects.

The level of said Oscillospira spp. in a test sample can be compared to the same bacteria in a control sample. A decreased level of Oscillospira spp. is related to a diagnosis that the test sample is from a subject suffering from a dysbiosis of Oscillospira spp., such as one associated with Irritable Bowel Syndrome.

As used herein, the level of Oscillospira bacteria in a test sample is increased when it is significantly higher than the level of said Oscillospira bacteria in a control sample. It is also considered increased when the level of Oscillospira bacteria in the test sample is at least 1-fold, 2-fold, 5-fold, 10-fold, 100-fold, 1000-fold or higher than the numbers of Oscillospira bacteria in the control sample.

As used herein, the numbers or level of Oscillospira bacteria in a test sample is decreased when it is significantly lower than the level of said Oscillospira bacteria in a control sample. It is also considered decreased when the level of Oscillospira bacteria in the test sample is at least 1-fold, 2-fold, 5-fold, 10-fold, 100-foldor 1000-fold lower than the level of Oscillospira in the control sample.

The levels of the nucleic acid sequences in a test sample may be subjected to statistical and/or bioinformatical analysis to obtain analyzed data; and the analyzed data of said test sample may be compared to analyzed data from a control sample, to provide a diagnosis of whether the test sample is from a subject suffering from a dysbiosis of Oscillospira spp. and any related disorder such as Irritable Bowel Syndrome. For example, hybridization patterns on a micro-array comprising the nucleic acid sequences may be processed using statistical and/or bioinformatical analysis such as Principal Component Analysis (PCA) and/or Redundancy Analysis (RDA). The analyzed data may then be compared to analyzed data from a control sample which has been subject to the same statistical and/or bioinformatical analysis.

Significance Analysis of Microarrays (SAM) can be used in comparing the levels of said three or more nucleic acid sequence from said test sample with the levels of nucleic acid sequence from a control sample. The person skilled in the art is capable of performing SAM analysis. SAM analysis is described in detail by Tusher et al. (Proc Natl Acad Sci USA, 2001, vol 98:5116-5121).

Prediction Analysis of Microarray (PAM) can be used in comparing the levels of said nucleic acid sequence from said test sample with the levels of nucleic acid sequence from a control sample. The person skilled in the art is capable of performing PAM analysis. PAM analysis is described in detail by Tibshirani et al., (Proc Natl Acad Sci USA, 2002, vol 99:6567-6572).

Redundancy Analysis (RDA) can be used in comparing the levels of nucleic acid sequence from said test sample with the levels of nucleic acid sequence from a control sample. The person skilled in the art is capable of performing RDA analysis. RDA analysis is described in detail by Leps and Smilauer (2003. Cambridge University Press: Multivariate analysis of ecological 780 data using CANOCO).

### EXAMPLES

The following non-limiting examples embody certain aspects of the invention and are described with reference to the accompanying figures in which:

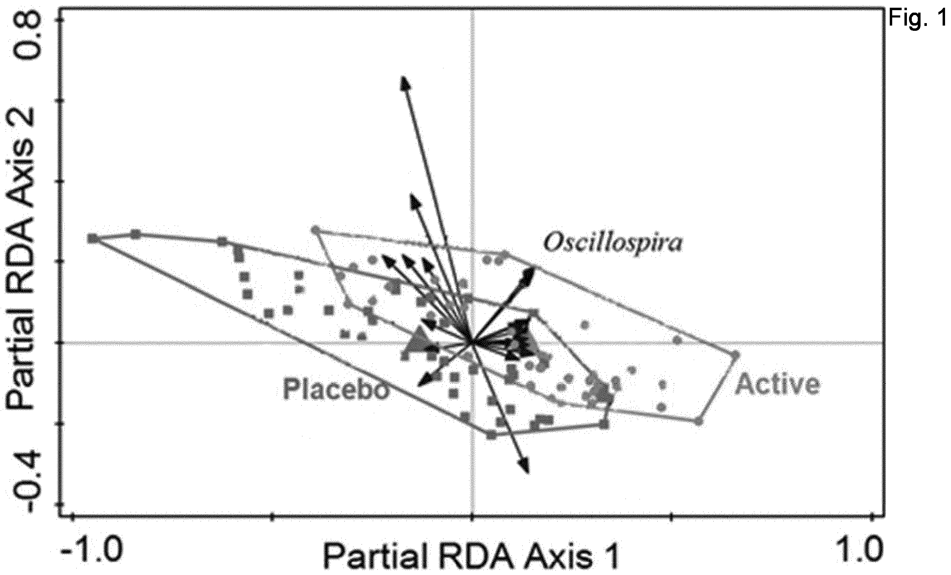

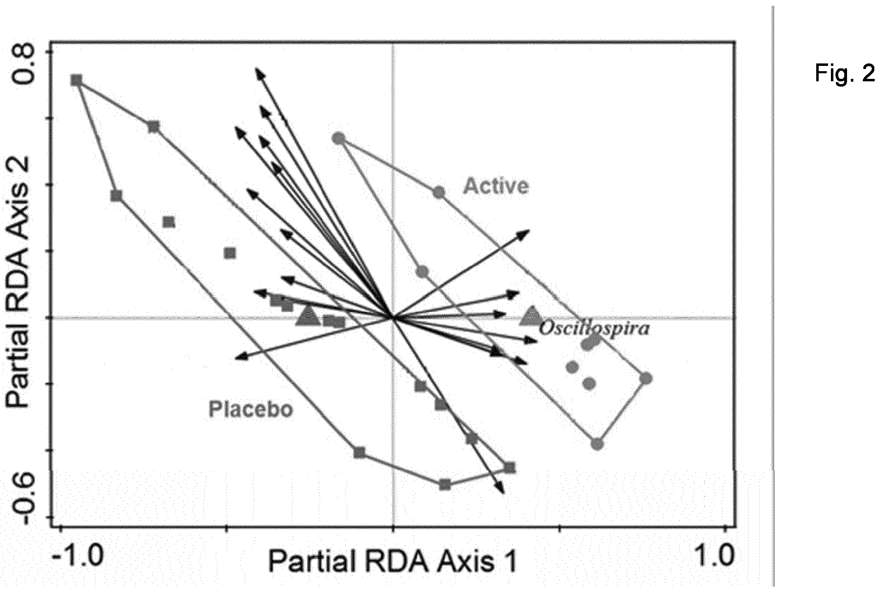

Figures 1 and 2 show the results of Redundancy Analysis (RDA).

RDA is a multivariable analysis technique that identifies the difference between sample groups that can be explained by a set of variables. In the RDAs in Figs 1 and 2, different sample properties *(e.g.* treatment group; treatment responders, etc.) are explained by microbiota compositions at the genus level.

In the RDA Figures 1 and 2 every dot is a sample. Black arrows represent genera and point in the direct of samples in which a genus is highly abundant. For discreet classes, the colours and shape of sample symbols indicate sample class. The coloured polygons ("envelopes") enclose all samples in a class. Large triangles are centroids ("average sample" of a class).
Fig. 1 Redundancy Analysis (RDA) of 93 samples showed that 4 weeks of treatment with *Lactobacillus plantarum* resulted in a different microbiota composition in the (active = circle symbol) group that received the *Lactobacillus plantarum* treatment (10¹⁰CFU once daily capsule containing freeze-dried *Lactobacillus* plantarum bacteria and exipient powder) vs. the placebo group (= square symbol), with the active group showing increased numbers of Oscillospira spp.
Fig. 2 Redundancy Analysis of 24 samples from individuals who were determined to be "abdominal pain responders according to the EMA guideline" (*i*.*e*. felt ≥ 30% better than their mean baseline value for ≥ 50% of the treatment period). Oscillospira spp. levels are higher in the group that received the *Lactobacillus plantarum* treatment (active) than in the group that received placebo;

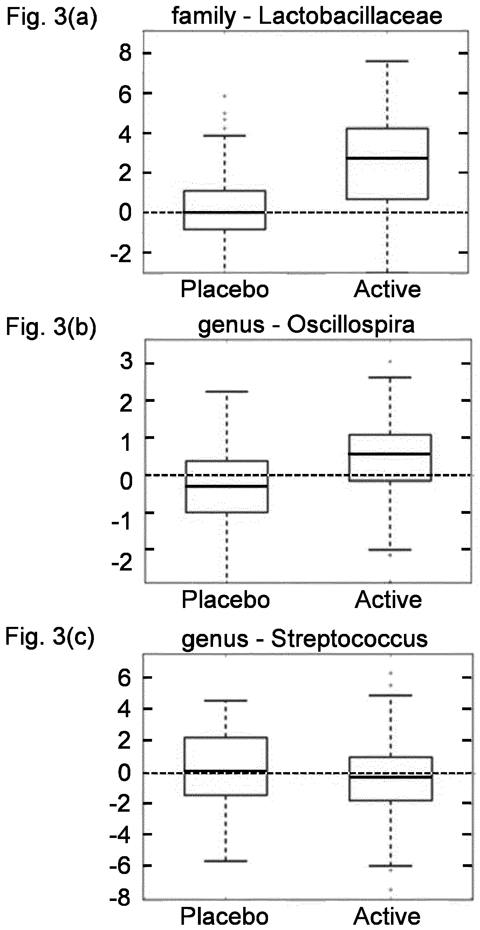

Fig. 3 shows the difference between "active" and "placebo" on a per-subject basis. The question the data addresses is: for a specific taxon, is the change in abundance going from "before" to "after" treatment different for subjects in the "active" and "placebo" groups? The vertical axis: 2 log (after/before) where an increase on the vertical axis signifies an increase in abundance with time. A selective effect is revealed by the analysis: There is only a significant difference in the first two boxplots: 3(a) *Lactobacillaceae* reflecting the *Lactobacillus plantarum* administered to subjects; and 3(b) Oscillospira spp. Streptococcus levels (3(c)) are unaffected by the treatment with *Lactobacillus plantarum.*

### EXAMPLE 1

**Clinical trial:** The study was a randomised, placebo-controlled, double-blind, parallel, intervention study with voluntary IBS out-patients with IBS according to Rome III criteria. After a screening visit and a 2 week run-in period patients with abdominal pain score between 3-6 on a 0-10 point Likert scale with a frequency of at least two days a week were randomised to receive either *Lactobacillus plantarum* 299v (10¹⁰ CFU/capsule) or placebo capsules one capsule per day for 4 weeks. A faecal sample was taken at the end of the run-in period and at the end of the intervention period. A food and physical activity questionnaire was filled in at the randomisation visit and at the last visit.

**16S rRNA gene profiling:** Illumina 16S rRNA gene amplicon libraries were generated and sequenced. In short, barcoded amplicons from the V3-V4 region of 16S rRNA genes were generated using a 2-step PCR. 10-25 ng genomic (g)DNA was used as template for the first PCR with a total volume of 50 ul using the 341F (5'-CCTACGGGNGGCWGCAG-3', SEQ ID NO: 1) and the 805R (5'-GACTACHVGGGTATCTAATCC-3', SEQ ID NO: 2) specific 16S rRNA primers appended with Illumina adaptor sequences. PCR products were purified and the size of the PCR products were checked on a Bioanalyzer (Agilent) and quantified by fluorometric analysis. Purified PCR products were used for the 2nd PCR in combination with sample-specific barcoded primers (Nextera XT index kit, Illumina). Subsequently, PCR products were purified, checked on a Bioanalyzer (Agilent) and quantified, followed by multiplexing, clustering, and sequencing on an Illumina MiSeq with the paired-end (2x) 300 bp protocol and indexing. The sequencing run was analyzed with the Illumina CASAVA pipeline (v1.8.3) with demultiplexing based on sample-specific barcodes. The raw sequencing data produced was processed removing the sequence reads of too low quality (only "passing filter" reads were selected) and discarding reads containing adaptor sequences or PhiX control with an in-house filtering protocol. A quality assessment on the remaining reads was performed using the FASTQC quality control tool version 0.10.0.

**Bioinformatics:** Microbiota composition analysis: microbiota composition was determined using a modified Qiime 1.8 workflow. Sample clustering was done with the "pick_openreference_otus" workflow script (97% identity) against the 13.8 version of the GreenGenes database using usearch as clustering methods. Reference-based chimera removal was done with uchime.
- Multivariate statistics: redundancy analysis (RDA) was done with Canoco 5.0.
- Univariate statistics were done in Scientific Python, using non-parametric tests (Mann-Whitney and Kruskal Wallis for unpaired data, Wilcoxon signed rank for paired data).

### RESULTS

Both fiber and Bristol Stool Scale (BSS) Score were shown to be confounders and were corrected for in the analyses. Feces samples without a corresponding BSS Score for that day were therefore removed from the analysis.

A redundancy analysis (RDA) revealed a trend towards a differential microbiota composition in the placebo group vs the active group at endpoint. The univariate analysis showed that the abundance of the genus Oscillospira was significantly higher in the active group at endpoint after correction for multiple testing.

According to the European Medicines Agency (EMA) Guidelines on the evaluation of medical products for the treatment of irritable bowel syndrome (IBS) dated 25 September 2014, an IBS patient is classified as a responder for abdominal pain if for at least 50% of the treatment time they exhibit an abdominal pain score which has improved at least 30% compared to their baseline score.

Fig. 1 analysis of 93 samples showed that after weeks of treatment with *Lactobacillus plantarum* (10¹⁰CFU per day) resulted in a different microbiota composition in the group that received the *Lactobacillus plantarum* treatment (active) vs. the placebo group, with the active group showing increased numbers of Oscillospira spp.
Fig. 2 analysis of 24 sample from responders for abdominal pain according to the EMA guidelines (*i.e.they* felt = 30% better than their mean baseline value for ≥ 50% of the treatment period). Oscillospira spp. levels are higher in the group that received the *Lactobacillus plantarum* treatment (active) than in the group that received placebo;
Fig. 3 shows the difference between "active" and "placebo" on a per-subject basis, pairing "before" with "after" data. The question the data addresses is: for a specific taxon, is the change in abundance going from "before" to "after" treatment difference for subjects in the "active" and "placebo" groups? A selective effect is revealed by the analysis: There is only a significant difference after correction for multiple testing in the first two boxplots: 3(a) *Lactobacillaceae* reflecting the *Lactobacillus plantarum* administered to subjects; and 3(b) Oscillospira spp. Streptococcus levels (3(c)) are unaffected by the treatment with *Lactobacillus plantarum.*

Overall, the present results show that treatment with *Lactobacillus plantarum* is effective to increase the numbers of Oscillospira spp. in a subject. Further, the results show that increased numbers of Oscillospira are associated with a pain response benefit in IBS patients treated with *Lactobacillus plantarum.* Hence, the invention should have application in the treatment and/or prophylaxis of a wide range of other disorders and diseases that have been associated with low levels of Oscillospira, which is a predicted butyrate-producer, and/or with disorders and diseases that have been associated with a low abundance of butyrate-producers.

## Claims

1. At least one strain of *Lactobacillus plantarum* DSM 9843 for use in the treatment of abdominal pain in a subject suffering from irritable bowel syndrome (IBS), wherein the subject is **characterised by** having a decreased number of Oscillospira spp. in a test sample derived from said subject relative to a control sample derived from a healthy subject or from a pool of samples from healthy subjects, wherein the *Lactobacillus plantarum* DSM 9843 is administered in an amount of from 10⁸ - 10¹¹ CFU per daily dose, preferably 10¹⁰ CFU per daily dose to attain increased numbers of Oscillospira spp. in the subject.

2. The least one strain of *Lactobacillus plantarum* DSM 9843 for use according to any preceding claim, wherein the at least one strain of *Lactobacillus plantarum* DSM 9843 is administered orally.

3. The least one strain of *Lactobacillus plantarum* DSM 9843 for use according to any preceding claim, wherein the at least one strain of *Lactobacillus plantarum* 9843 is provided in a composition in combination with an excipient or carrier material.

4. The least one strain of *Lactobacillus plantarum* DSM 9843 for use according to Claim 3,
wherein the excipient is a pharmaceutically acceptable excipient.

5. The least one strain of *Lactobacillus plantarum* DSM 9843 for use according to Claim 3 or 4, wherein the carrier material is a human and/or animal food material.

6. The least one strain of *Lactobacillus plantarum* DSM 9843 for use according to any preceding claim, wherein the at least one strain of *Lactobacillus plantarum* DSM 9843 is provided as a composition in combination with a micronutrient, such as a mineral and/or vitamin.

7. The least one strain of *Lactobacillus plantarum* DSM 9843 for use according to any of Claims 3-6, wherein the composition is adapted for oral administration.

## Patentansprüche

1. Mindestens ein Stamm von Lactobacillus plantarum DSM 9843 zur Verwendung bei der Behandlung von Bauchschmerzen bei einem an Reizdarmsyndrom (IBS) leidenden Proband, wobei der Proband **gekennzeichnet ist durch** eine verringerte Anzahl von Oscillospira spp. in einem von dem Probanden stammenden Prüflos relativ zu einer Kontrollprobe, die von einem gesunden Probanden oder aus einem Pool von Proben von gesunden Probanden stammt, wobei das Lactobacillus plantarum DSM 9843 in einer Menge von 10⁸ - 10¹¹ KBE pro Tagesdosis, vorzugsweise 10¹⁰ KBE pro Tagesdosis, verabreicht wird, um eine erhöhte Anzahl von Oscillospira spp. in dem Probanden zu erreichen.

2. Mindestens ein Stamm von Lactobacillus plantarum DSM 9843 zur Verwendung nach einem der vorstehenden Ansprüche, wobei der mindestens eine Stamm von Lactobacillus plantarum DSM 9843 oral verabreicht wird.

3. Mindestens ein Stamm von Lactobacillus plantarum DSM 9843 zur Verwendung nach einem der vorstehenden Ansprüche, wobei der mindestens eine Stamm von Lactobacillus plantarum 9843 in einer Zusammensetzung in Kombination mit einem Hilfsstoff oder Trägermaterial bereitgestellt wird.

4. Mindestens ein Stamm von Lactobacillus plantarum DSM 9843 zur Verwendung nach Anspruch 3, wobei der Hilfsstoff ein pharmazeutisch verträglicher Hilfsstoff ist.

5. Mindestens ein Stamm von Lactobacillus plantarum DSM 9843 zur Verwendung nach Anspruch 3 oder 4, wobei das Trägermaterial ein Nahrungsmittelmaterial für Menschen und/oder Tiere ist.

6. Mindestens ein Stamm von Lactobacillus plantarum DSM 9843 zur Verwendung nach einem der vorstehenden Ansprüche, wobei der mindestens eine Stamm von Lactobacillus plantarum DSM 9843 als eine Zusammensetzung in Kombination mit einem Mikronährstoff, wie einem Mineral und/oder Vitamin, bereitgestellt wird.

7. Mindestens ein Stamm von Lactobacillus plantarum DSM 9843 zur Verwendung nach einem der Ansprüche 3 bis 6, wobei die Zusammensetzung zur oralen Verabreichung angepasst ist.

## Revendications

1. Au moins une souche de *Lactobacillus plantarum* DSM 9843 pour utilisation dans le traitement de douleurs abdominales chez un sujet souffrant du syndrome du côlon irritable (SCI), dans laquelle le sujet est **caractérisé par** une diminution du nombre d'Oscillospira spp. dans un test échantillon prélevé sur ledit sujet par rapport à un échantillon prélevé sur témoin un sujet sain ou à partir d'un pool d'échantillons provenant de sujets sains, dans laquelle le *Lactobacillus plantarum* DSM 9843 est administré en une quantité allant de 10⁸ à 10¹¹ UFC par dose journalière, de préférence 10¹⁰ UFC par dose journalière pour obtenir un nombre accru d'Oscillospira spp. chez le sujet.

2. L'au moins une souche de *Lactobacillus plantarum* DSM 9843 pour utilisation selon l'une quelconque revendication précédente, dans laquelle l'au moins une souche de *Lactobacillus plantarum* DSM 9843 est administrée par voie orale.

3. L'au moins une souche de *Lactobacillus plantarum* DSM 9843 pour utilisation selon l'une quelconque revendication précédente, dans laquelle l'au moins une souche *de Lactobacillus plantarum* 9843 est fournie dans une composition en combinaison avec un excipient ou un matériau porteur.

4. L'au moins une souche de *Lactobacillus plantarum* DSM 9843 pour utilisation selon la revendication 3, dans laquelle l'excipient est un excipient pharmaceutiquement acceptable.

5. L'au moins une souche de *Lactobacillus plantarum* DSM 9843 pour utilisation selon la revendication 3 ou 4, dans laquelle le matériau porteur est un matériau alimentaire pour humains et/ou animaux.

6. L'au moins une souche de *Lactobacillus plantarum* DSM 9843 pour utilisation selon l'une quelconque revendication précédente, dans laquelle l'au moins une souche de *Lactobacillus plantarum* DSM 9843 est fournie en tant que composition en combinaison avec un micronutriment, tel qu'un minéral et/ou une vitamine.

7. L'au moins une souche de *Lactobacillus plantarum* DSM 9843 pour utilisation selon l'une quelconque des revendications 3 à 6, dans laquelle la composition est adaptée pour une administration orale.
